# EUROPEAN PATENT APPLICATION

(11) **EP 2 949 274 A1**
(43) Date of publication of application: **02.12.2015**
(21) Application number: 13872492.7
(22) Date of filing: 13.09.2013
(51) Int. Cl.: A61B 17/00

(54) **HEART TREATMENT DEVICE**

(30) Priority: 23.01.2013 JP 2013010115
(71) Applicant: Olympus Corporation, Shibuya-ku Tokyo 151-0072 (JP)
(72) Inventor: SUGAHARA, Michihiro, Tokyo 151-0072 (JP)
(74) Representative: Schicker, Silvia
(86) International application number: PCT/JP2013/074816
(87) International publication number: WO 2014/115370

(57) **Abstract**

During heart treatment performed via a port, the heart is moved without impeding the circulatory dynamics in the cardiac sac. Provided is a cardiac treatment apparatus (1) including: an annular portion equipped with a wire that can be deformed between a contracted shape that allows insertion into the cardiac sac via a tubular port (9) disposed passing from a body surface to the pericardium (B) and an annular expanded shape in which the annular portion is expanded in the cardiac sac, is fitted onto the outer circumference of the heart (A) from a ventricular apex (Al) side, and is located at a position for surrounding the outer circumference of the heart (A); and a pulling portion that exerts a pulling force on the annular portion such that the annular portion presses a sidewall of the heart (A), with the annular portion having been located at the position for surrounding the heart (A).

## Description

### {Technical Field}

The present invention relates to a cardiac treatment apparatus.

### {Background Art}

In the related art, there is a known organ manipulator equipped with means for holding a beating heart during surgery (for example, see PTL 1).

This organ manipulator is attached by suction to the heart which is exteriorized through a thoracotomy to turn the heart about the ventricular apex.

### {Citation List}

### {Patent Literature}

{PTL 1} Publication of Japanese Patent No. 4503900 {Summary of Invention}

### {Technical Problem}

Because the organ manipulator of PTL 1 is attached to the ventricular apex by suction and rotates the ventricular apex in a state in which, during open chest surgery, contraction of the lung is controllable, and the membrane structure is incised to put the heart into an exteriorized state in which the heart has an extremely high degree of freedom, the organ manipulator is acceptable even though it has a complicated structure and is large.

However, in a case in which treatment is performed via a port, such as a sheath or a trocar, a large organ manipulator such as that disclosed in PTL 1 cannot be used. Furthermore, in this case, the heart is at a more normal anatomical location; therefore, rotational movement about the ventricular apex is not always the best moving method.

The present invention has been made in view of the above-described circumstances, and an object thereof is to provide a cardiac treatment apparatus capable of moving the heart without impeding the circulatory dynamics in the cardiac sac during heart treatment performed the via a port.

### {Solution to Problem}

In order to achieve the above-described object, the present invention provides the following solutions.

According to one aspect, the present invention provides a cardiac treatment apparatus including: an annular portion including a wire that can be deformed between a contracted shape that allows insertion into a cardiac sac via a tubular port disposed passing from a body surface to a pericardium and an annular expanded shape in which the annular portion is expanded in the cardiac sac, is fitted onto the outer circumference of the heart from a side of a ventricular apex, and is located at a position for surrounding the outer circumference of the heart; and a pulling portion that exerts a pulling force on the annular portion such that the annular portion presses a sidewall of the heart, with the annular portion having been located at the position for surrounding the heart.

According to this aspect, the tubular port that penetrates from the body surface to the pericardium is prepared; the annular portion in a contracted shape is made to pass through the port, is deformed into an annular expanded shape in the cardiac sac, is fitted onto the outer circumference of the heart from the ventricular apex side, and is located at a position for surrounding the outer circumference of the heart; and the pulling portion exerts the pulling force on the annular portion, thereby making it possible to impose a force on the sidewall of the heart to move the ventricular apex about the mediastinum. Accordingly, it is possible to move the heart without impeding the circulatory dynamics in the cardiac sac during heart treatment performed via the port.

In the above-described aspect, the annular portion may be deformable into an expanded shape that does not impede beating of the heart.

By doing so, even when the annular portion expanded in the cardiac sac is located at a position for surrounding the outer circumference of the heart, beating of the heart is not impeded; therefore, the heart can be moved with even less impediment to the circulatory dynamics.

In the above-described aspect, the annular portion may include, at a portion thereof for surrounding the heart, an expandable/contractable coiled part.

By doing so, even when the annular portion is closely located at a position for surrounding the outer circumference of the heart, dilation and contraction of the heart when beating can be allowed by expansion and contraction of the coiled part, and the heart can be moved with even less impediment to the circulatory dynamics.

In the above-described aspect, the annular portion may include, at a portion thereof for surrounding the heart, an expandable/contractable balloon that is located between the wire, which constitutes the annular portion, and the heart.

By doing so, even when the annular portion is closely located at a position for surrounding the outer circumference of the heart, dilation and contraction of the heart when beating can be allowed by expansion and contraction of the balloon, and the heart can be moved with even less impediment to the circulatory dynamics.

In the above-described aspect, the annular portion may be provided with a membranous portion that can be deformed into a shape that allows the membranous portion to pass through the port and that can be deformed to be expanded into a shape in which the membranous portion is brought into close contact with the ventricular apex when the annular portion is located at the position for surrounding the outer circumference of the heart.

By doing so, when the annular portion is located at a position for surrounding the outer circumference of the heart, the membranous portion is brought into close contact with the ventricular apex; therefore, when the pulling force is exerted on the annular portion by the pulling portion, it is possible to exert, in addition to the pressing force exerted on the heart by the annular portion, the pulling force on the ventricular apex by means of the friction between the membranous portion and the ventricular apex. As a result, it is possible to exert the pulling force on the heart in a wider area and to move the heart while preventing a localized pressure from being imposed on the heart.

In the above-described aspect, it is possible to further include a suction portion that can be deformed into a shape which allows the suction portion to pass through the port, that is located at a position where the suction portion is attached by suction to the sidewall of the heart having been moved by being pressed by the annular portion, and that can fix the position of the heart.

By doing so, the suction portion can be attached by suction to the heart, which has been moved, to fix the heart, and a situation in which a strong pulling force is exerted on the heart need not be maintained.

According to another aspect, the present invention provides a cardiac treatment apparatus including: an annular portion including a wire that can be deformed between a contracted shape that allows insertion into a cardiac sac via a tubular port disposed passing from a body surface to a pericardium and an annular expanded shape in which the annular portion is expanded in the cardiac sac, is fitted onto the outer circumference of the heart from a side of a ventricular apex, and is located at a position for surrounding the outer circumference of the heart; a suction portion that is provided in the annular portion, that can be deformed into a shape which allows the suction portion to pass through the port, that is located at a position where the suction portion reaches the ventricular apex while the annular portion is being fitted onto the outer circumference of the heart from the side of the ventricular apex, and that is attached to the ventricular apex by suction; and a pulling portion that pulls the suction portion in a direction intersecting a straight line connecting a mediastinum and the ventricular apex.

According to this aspect, when the annular portion is fitted onto the heart from the ventricular apex side, is located at a position for surrounding the outer circumference of the heart, and is moved along the surface of the heart, the suction portion, which is provided in the annular portion, reaches the ventricular apex. Accordingly, the suction portion can be easily positioned at the position corresponding to the ventricular apex. Therefore, the suction portion is moved to be attached to the ventricular apex by suction, and the pulling portion is made to exert the pulling force, thereby making it possible to pull the suction portion to exert the pulling force on the ventricular apex and to move the ventricular apex about the mediastinum.

### {Advantageous Effects of Invention}

According to the present invention, an advantageous effect is afforded in that it is possible to move the heart without impeding the circulatory dynamics in the cardiac sac during heart treatment performed via a port.

### {Brief Description of Drawings}

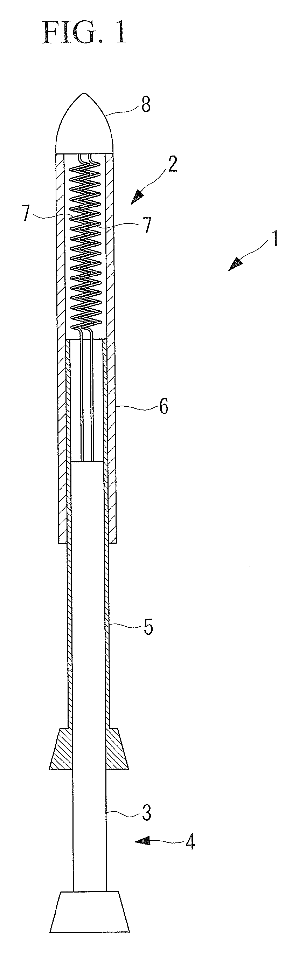

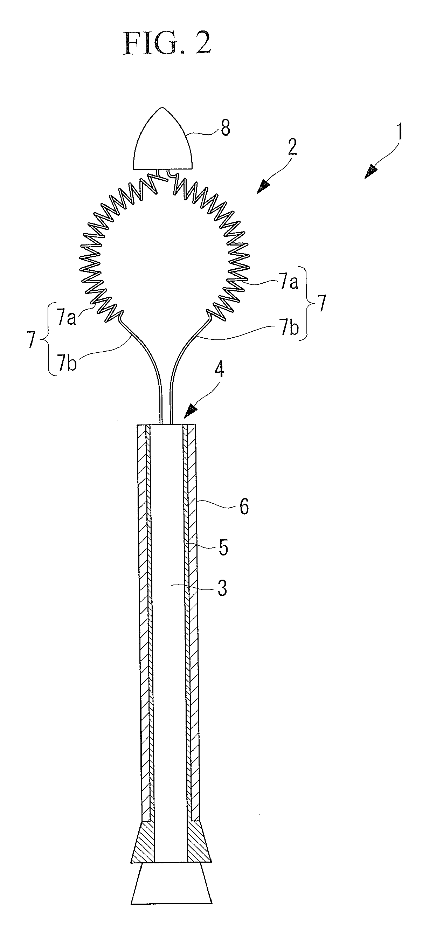

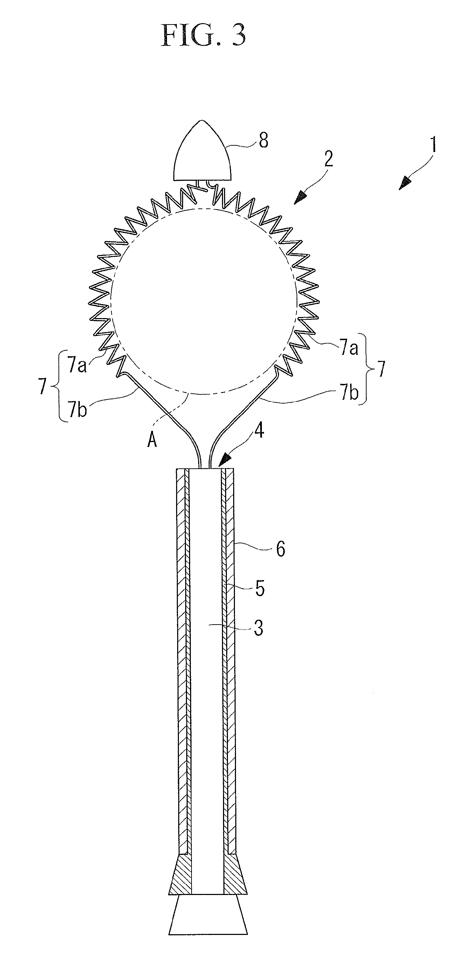

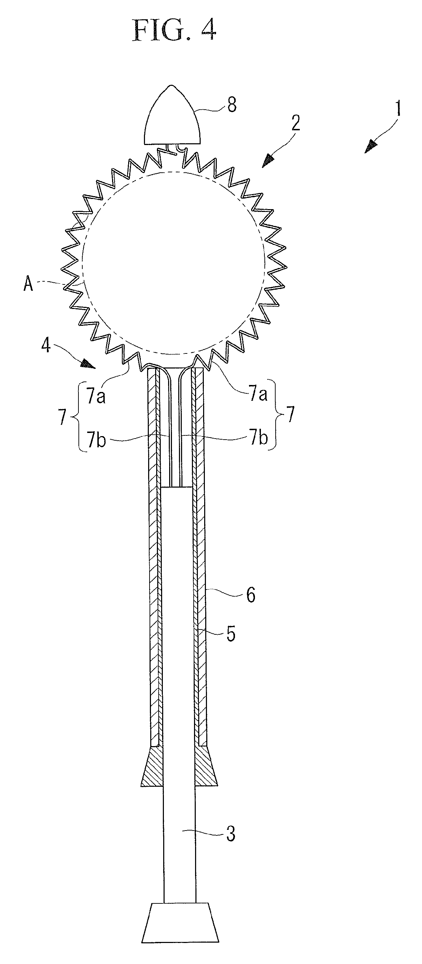

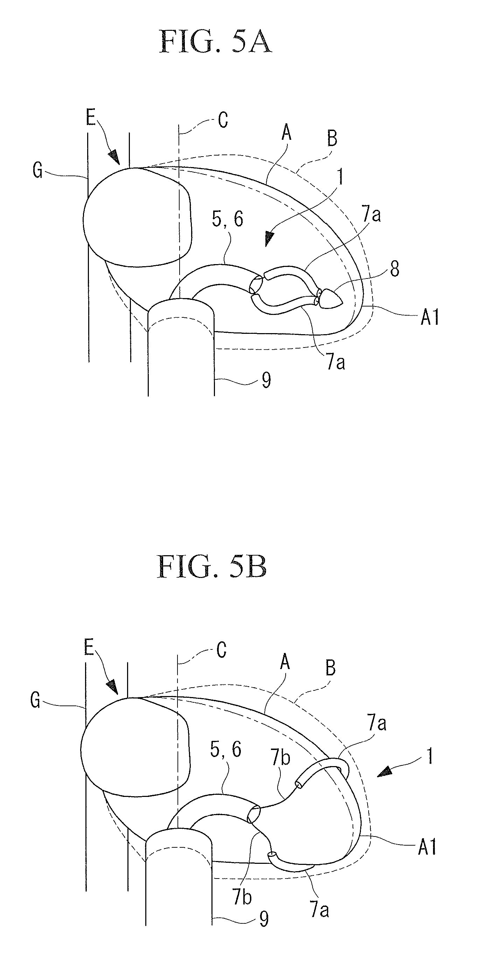

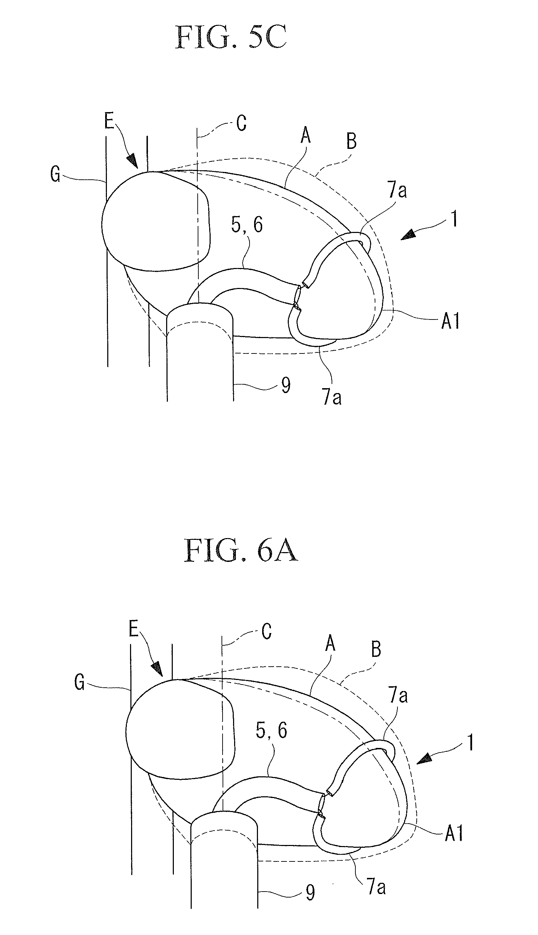

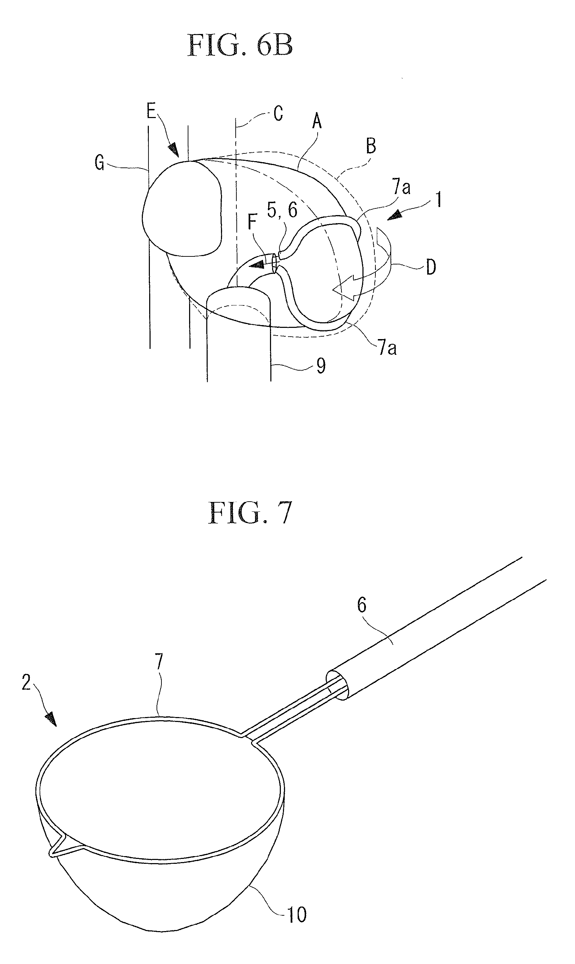

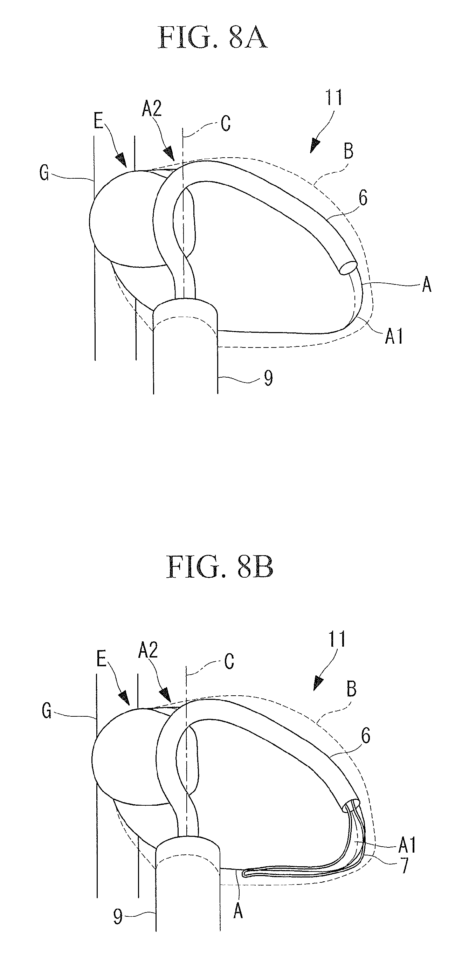

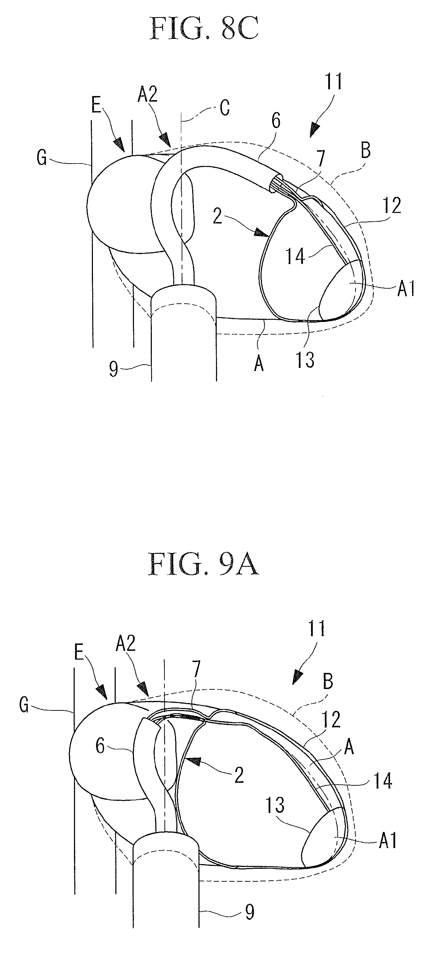

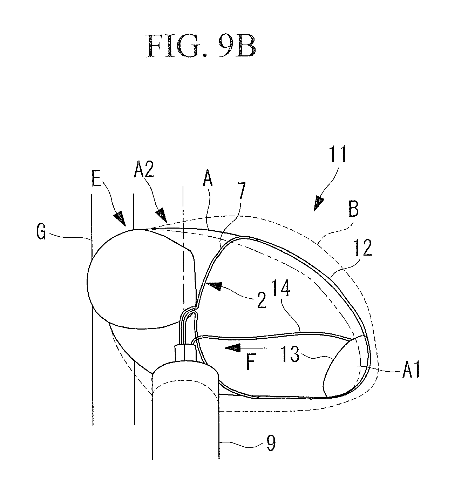

{Fig. 1} Fig. 1 is a longitudinal sectional view showing a cardiac treatment apparatus according to one embodiment of the present invention.
{Fig. 2} Fig. 2 is a longitudinal sectional view showing a state in which an annular portion is exposed from a storage sheath, in the cardiac treatment apparatus shown in Fig. 1.
{Fig. 3} Fig. 3 is a longitudinal sectional view showing a state in which the heart is located inside the exposed annular portion, in the cardiac treatment apparatus shown in Fig. 1.
{Fig. 4} Fig. 4 is a longitudinal sectional view showing a state in which the heart located inside the annular portion is tightly gripped by the annular portion, in the cardiac treatment apparatus shown in Fig. 1.
{Fig. 5A} Fig. 5A is a view for explaining treatment performed using the cardiac treatment apparatus shown in Fig. 1 and a view showing a state in which the annular portion is exposed in the cardiac sac.
{Fig. 5B} Fig. 5B is a view for explaining the treatment performed using the cardiac treatment apparatus shown in Fig. 1 and a view showing a state in which the annular portion is hooked on a portion of the heart in the vicinity of a ventricular apex.
{Fig. 5C} Fig. 5C is a view for explaining the treatment performed using the cardiac treatment apparatus shown in Fig. 1 and a view showing a state in which the heart is tightly gripped by the annular portion.
{Fig. 6A} Fig. 6A is a view for explaining the treatment performed using the cardiac treatment apparatus shown in Fig. 1 and is a view similar to Fig. 5C.
{Fig. 6B} Fig. 6B is a view for explaining the treatment performed using the cardiac treatment apparatus shown in Fig. 1 and is a view showing a state in which the heart is rotated by pulling the annular portion.
{Fig. 7} Fig. 7 is a perspective view showing a first modification of the cardiac treatment apparatus shown in Fig. 1.
{Fig. 8A} Fig. 8A is a view for explaining treatment according to another modification of the cardiac treatment apparatus shown in Fig. 1 and is a view showing a state in which the cardiac treatment apparatus is introduced into the cardiac sac.
{Fig. 8B} Fig. 8B is a view for explaining the treatment according to the modification of the cardiac treatment apparatus shown in Fig. 1 and is a view showing a state in which a wire etc. are pushed out from the storage sheath.
{Fig. 8C} Fig. 8C is a view for explaining the treatment according to the modification of the cardiac treatment apparatus shown in Fig. 1 and is a view showing a state in which the annular portion is fitted onto the heart, and a basket-shaped member covers the ventricular apex.
{Fig. 9A} Fig. 9A is a view for explaining the treatment according to the modification of the cardiac treatment apparatus shown in Fig. 1 and is a view showing a state in which the annular portion is pulled up to bring a suction portion into close contact with the ventricular apex.
{Fig. 9B} Fig. 9B is a view for explaining the treatment according to the modification of the cardiac treatment apparatus shown in Fig. 1 and is a view showing a state in which the suction portion is attached to the ventricular apex by suction to exert a pulling force.

### {Description of Embodiments}

A cardiac treatment apparatus 1 according to a first embodiment of the present invention will be described below with reference to the drawings.

As shown in Fig. 1, the cardiac treatment apparatus 1 of this embodiment includes, at the distal end thereof, an annular portion 2 made of wires and includes, at the base end thereof, a coil snare 4 that has a columnar manipulation portion (pulling portion) 3, a cylindrical auxiliary sheath 5 into which the manipulation portion 3 of the coil snare 4 is fitted so as to be capable of moving in an axial direction, and a cylindrical storage sheath 6 into which the auxiliary sheath 5 is also fitted so as to be capable of moving in the axial direction.

The annular portion 2 of the coil snare 4 has two wires 7 that can be deformed between a contracted shape in which accommodation in the storage sheath 6 is possible, as shown in Fig. 1, and an expanded shape in which the wires 7 are released from the storage sheath 6 and expanded in a circular shape, as shown in Fig. 2. The two wires 7 are connected to a distal-end member 8 that is formed so as to be tapered at the distal end.

The wires 7 have coiled parts 7a that are disposed closer to the distal-end member 8 and non-coiled parts 7b that are disposed at the base ends of the coiled parts 7a. With the coiled parts 7a, the two wires 7 can be located so as to be in close contact with an outer surface of a heart A and so as to surround the outer circumference thereof, by making the coiled parts 7a expand and contract according to the circumferential length of the heart A to be inserted therebetween, as shown in Fig. 3.

As shown in Fig. 3, even though the heart A beats, causing it to dilate and contract, with the coiled parts 7a surrounding the outer circumference of the heart A, the coiled parts 7a are expanded and contracted, thereby following shape changes of the heart A to maintain the coiled parts 7a in close contact with the outer surface of the heart A.

The manipulation portion 3 of the coil snare 4, the auxiliary sheath 5, and the storage sheath 6 are made of flexible materials and can be bent as desired.

The operation of the thus-configured cardiac treatment apparatus 1 of this embodiment will be described below.

To move the heart A using the cardiac treatment apparatus 1 of this embodiment, as shown in Figs. 5A to 5C, a port 9 that penetrates, in the vicinity of substantially a midline C, body tissue and a pericardium B from below the ensiform cartilage and that opens into the cardiac sac is provided, and the cardiac treatment apparatus 1 is introduced into the cardiac sac via the port 9.

Specifically, as shown in Fig. 1, the storage sheath 6 is moved forward with respect to the coil snare 4 until it reaches the distal-end member 8, thus accommodating the annular portion 2 of the coil snare 4 in the storage sheath 6. Accordingly, the cardiac treatment apparatus 1 becomes a tubular member having an outer surface with low roughness, thus facilitating insertion into the cardiac sac via the port 9.

Because the cardiac treatment apparatus 1 has the tapered distal-end member 8 at the distal end, when introduced into the cardiac sac, the cardiac treatment apparatus 1 is curved along the nearby heart A and pericardium B and is moved forward along a gap between the heart A and the pericardium B in a direction toward a ventricular apex A1. Then, when the distal-end member 8 is located in the vicinity of the ventricular apex A1, the auxiliary sheath 5 and the storage sheath 6 are moved backward with respect to the manipulation portion 3 of the coil snare 4 to expose the annular portion 2 of the coil snare 4. Accordingly, the coiled parts 7a of the annular portion 2 released from the storage sheath 6 are expanded in a circular shape, as shown in Fig. 5A.

In this state, when the coil snare 4 is moved farther forward with respect to the auxiliary sheath 5, as shown in Fig. 3 and Fig. 5B, the non-coiled parts 7b of the annular portion 2 are also exposed and expanded in a large circular shape, and thus the expanded annular portion 2 is hooked on a ventricular apex A1 to locate the heart A inside the annular portion 2. Then, from this state, the auxiliary sheath 5 and the storage sheath 6 are moved farther forward with respect to the coil snare 4, thereby accommodating the non-coiled parts 7b of the annular portion 2 in the auxiliary sheath 5 and extending the coiled parts 7a due to the tension thereof, as shown in Fig. 4 and Fig. 5C.

Accordingly, the annular portion 2 of the coil snare 4 is located so as to surround the outer circumference of the heart A in the vicinity of the ventricular apex A1 and is brought into close contact with the outer surface of the heart A due to the elasticity of the coiled parts 7a.

After that, from a state shown in Fig. 6A, a pulling force F is exerted in a direction in which the manipulation portion 3 of the coil snare 4 is pulled out from the port 9, thereby pressing the annular portion 2 against the outer surface of the heart A to exert the pulling force F on the heart A. Accordingly, as shown by an arrow D in Fig. 6B, the heart A is rotated about a mediastinum E so as to move the ventricular apex A1, thereby making it possible to move a side surface on the dorsal side of the heart A to the chest side.

An upper portion of the heart A is fixed to the mediastinum E, which is connected to a dorsal midline, and a lower portion thereof is not connected to anything so as not to impede complicated movements for beating, such as dilation/contraction and swiveling, thus having a high degree of freedom. The upper portion of the heart A, which is connected to the mediastinum E, is a place where the vena cava G interacts with the right atrium, and the tissue strength, in particular the thickness thereof, is lower than that of the aorta and the other portions of the heart A. Thus, the upper portion of the heart A is a portion where damage around the heart A frequently occurs due to sudden body movement caused by a car accident, for example.

Therefore, by rotating the heart A about the mediastinum E, it is possible to move the heart A while preventing the connected portion between the vena cava G and the right atrium from being pulled or from being compressed, thus preventing beating from being affected.

Furthermore, according to the cardiac treatment apparatus 1 of this embodiment, because the coiled parts 7a, which are provided in the annular portion 2, are located so as to surround the outer circumference of the heart A to be brought into close contact therewith and exert the pulling force F on the heart A, even when the heart A dilates or contracts through beating, the coiled parts 7a can be expanded and contracted to follow the dilation and contraction. Therefore, the heart A can be easily rotated by exerting the pulling force F thereon without impeding beating of the heart A.

Furthermore, after treatment for the side surface on the dorsal side of the heart A is completed, in order to remove the cardiac treatment apparatus 1 of this embodiment from the heart A and pull out it from the cardiac sac via the port 9, the manipulation portion 3 is moved forward to return the heart A to the original position, and the auxiliary sheath 5 and the storage sheath 6 are moved backward with respect to the coil snare 4 to expose the non-coiled parts 7b. Accordingly, the annular portion 2 exposed from the storage sheath 6 is increased in length, thus easing the constricted state of the heart A caused by the coiled parts 7a.

Then, the whole cardiac treatment apparatus 1 is moved farther forward, thus separating the coiled parts 7a from the surface of the heart A. After the separation, the storage sheath 6 is moved forward with respect to the coil snare 4 until it reaches the distal-end member 8, thereby accommodating the annular portion 2 in the storage sheath 6 and forming a cylindrical shape having no roughness on the surface thereof. Accordingly, the cardiac treatment apparatus 1 can be easily pulled out from the cardiac sac via the port 9, without involving the surrounding tissue.

In the cardiac treatment apparatus 1 of this embodiment, the annular portion 2 has the coiled parts 7a, which are formed by winding the wires 7, thereby making it possible to exert the pulling force F while expanding and contracting to follow beating of the heart A. Instead of this, the coiled parts 7a can be replaced with a balloon (not shown). With the balloon, it is possible to follow dilation and contraction caused by beating of the heart A, due to the elastic deformation of the balloon, and to transfer the pulling force F to the heart A without impeding beating of the heart A, in the same way as the coiled parts 7a.

The balloon may have a sufficient length to surround the entire outer circumference of the heart A or may be provided partially in the circumferential direction. Alternatively, a plurality of balloons may be provided at intervals in the circumferential direction.

Furthermore, in the cardiac treatment apparatus 1 of this embodiment, as shown in Fig. 7, it is also possible to configure the annular portion 2 by forming a single wire 7 connected to the manipulation portion 3 into an annular shape and to attach a sac-like membranous member 10 to the annular portion 2. The membranous member 10 can be accommodated in the storage sheath 6 in the contracted state together with the wire 7, and, when exposed from the storage sheath 6, the membranous member 10 is expanded into an expanded shape for bringing it into precise close contact with the ventricular apex A1.

The membranous member 10 is made of a material such as plastic mesh, metallic mesh, medical gauze, a water-absorbing polymer, or the like, and, when placed on the ventricular apex A1, the membranous member 10 is brought into close contact with the surface of the ventricular apex A1 due to friction or water absorption caused by capillary action. Accordingly, even when the heart A is not tightly gripped by the wire 7, the pulling force F applied to the wire 7 can be exerted on the heart A due to the close contact between the membranous member 10 and the ventricular apex A1, thus making it possible to easily rotate the heart A.

Although a description has been given of an example case in which the membranous member 10 is naturally expanded when the wire 7 is expanded, instead of this, it is possible to provide another wire (not shown) for expanding the membranous member 10 to reliably form a cup shape for easily bringing it into close contact with the ventricular apex A1. Furthermore, the annular portion 2, which is made of the wire 7, may be pressed and deformed by using the storage sheath 6 to tightly grip the heart A for achieving close contact.

Furthermore, in this embodiment, a suction pad (not shown) to be expanded in the vicinity of the outlet of the port 9 in the cardiac sac may be prepared in the vicinity of the outlet of the port 9 in the cardiac sac, and the suction pad may be attached by suction to the outer surface of the heart A having been rotated by the coil snare 4 etc., thereby maintaining the position of the heart A at the rotated position. Accordingly, a dorsal-side portion of the heart A having been moved to the chest side can be easily treated by using a treatment tool introduced via the port 9 or another port.

Next, a cardiac treatment apparatus 11 according to a second embodiment of the present invention will be described with reference to the drawings.

In the description of this embodiment, identical reference signs are assigned to portions having the same configurations as those of the above-described cardiac treatment apparatus 1 of the first embodiment, and a description thereof will be omitted.

As shown in Fig. 8C, the cardiac treatment apparatus 11 of this embodiment includes: the annular portion 2 that is made of the wire 7 whose size is sufficient to loosely surround the outer circumference of the heart A; a sac-like basket-shaped member 12 that covers the inside of the annular portion 2; a suction pad 13 that has a suction port at the apex of the basket-shaped member 12 on the inner side of the basket-shaped member 12; and a tube 14 that supplies a negative pressure to the suction pad 13 and also exerts the pulling force F on the suction pad 13.

The basket-shaped member 12 has roughly the same shape as the heart A so as to cover the heart A from the ventricular apex A1 side. Furthermore, the basket-shaped member 12 is made of a transparent polymer or a framework structure, a mesh structure, or the like that has many gaps so as not to impede observation such that observation and treatment can be performed through the basket-shaped member 12.

The basket-shaped member 12 and the suction pad 13 are accommodated in the storage sheath 6 in a contracted shape together with the wire 7 and are expanded by being released from the storage sheath 6.

In order to move the heart A by using the thus-configured cardiac treatment apparatus 11 of this embodiment, as shown in Fig. 8A, when the storage sheath 6, with the wire 7, the basket-shaped member 12, and the suction pad 13 having been accommodated therein, is introduced into the cardiac sac via the port 9, the storage sheath 6 advances to a head portion A2 of the heart A along the space between the heart A and the pericardium B and then goes down to the ventricular apex A1 in an inverted U-shaped curve through the head portion A2.

From the state in Fig. 8A, in which the storage sheath 6 has reached the vicinity of the ventricular apex A1, as shown in Figs. 8B and 8C, the wire 7, the basket-shaped member 12, and the suction pad 13 are pushed out from the storage sheath 6 and are expanded. Then, after the annular portion 2 having a shape capable of loosely surrounding the heart A is hooked on the heart A, the whole storage sheath 6 is moved backward such that the distal end of the storage sheath 6 is moved while following the same path in the direction opposite to the direction in which it was introduced, thereby pulling up the annular portion 2 from the ventricular apex A1 side to the head portion A2 side.

Accordingly, as shown in Fig. 8C, the sac-like basket-shaped member 12, which covers the inside of the annular portion 2, is located so as to cover the outer circumference of the heart A from the ventricular apex A1 side. Then, as shown in Fig. 9A, with the annular portion 2 made to come closest to the head portion A2, the suction pad 13, which is located inward at the apex of the basket-shaped member 12, is brought into close contact with the ventricular apex A1.

In this state, a negative pressure is supplied to the suction pad 13 via the tube 14, thereby attaching the suction pad 13 to the ventricular apex A1 by suction, and the tube 14 is pulled to exert the pulling force F on the suction pad 13, thereby making it possible to exert the pulling force F on the ventricular apex A1 via the suction pad 13 and to rotate the heart A about the mediastinum E.

In this embodiment, because the annular portion 2 has a size sufficient to loosely surround the heart A and is used to make the suction pad 13 come close to the ventricular apex A1, the annular portion 2 neither binds the heart A nor impedes beating of the heart A.

As a method of moving the whole storage sheath 6 backward such that the distal end thereof is moved while following the same path in the direction opposite to the direction in which it was introduced to the vicinity of the ventricular apex A1, it is possible to use, for example, a method in which balloons that are dilated between the heart A and the pericardium B, thereby fixing respective portions of the storage sheath 6 so as not to be moved, are provided at the respective portions of the storage sheath 6, and the storage sheath 6 is moved while switching between dilation and contraction of the balloons.

Furthermore, the storage sheath 6 may be formed to have a double-tube shape, and, when the storage sheath 6 is introduced into the cardiac sac to the vicinity of the head portion A2 of the heart A, only an internal storage sheath can be introduced toward the ventricular apex A1. In this case, an external storage sheath can be fixed in the vicinity of the head portion A2.

### {Reference Signs List}

- A1: ventricular apex
- A: heart
- B: pericardium
- F: pulling force
- 1, 11: cardiac treatment apparatus
- 2: annular portion
- 3: manipulation portion (pulling portion)
- 7: wire
- 7a: coiled parts
- 9: port
- 10: membranous portion
- 13: suction portion
- 14: tube (pulling portion)

## Claims

1. A cardiac treatment apparatus comprising:
an annular portion including a wire that can be deformed between a contracted shape that allows insertion into a cardiac sac via a tubular port disposed passing from a body surface to a pericardium and an annular expanded shape in which the annular portion is expanded in the cardiac sac, is fitted onto the outer circumference of the heart from a side of a ventricular apex, and is located at a position for surrounding the outer circumference of the heart; and
a pulling portion that exerts a pulling force on the annular portion such that the annular portion presses a sidewall of the heart, with the annular portion having been located at the position for surrounding the heart.

2. A cardiac treatment apparatus according to claim 1, wherein the annular portion can be deformed into an expanded shape that does not impede beating of the heart.

3. A cardiac treatment apparatus according to claim 2, wherein the annular portion includes, at a portion thereof for surrounding the heart, an expandable/contractable coiled part.

4. A cardiac treatment apparatus according to claim 2, wherein the annular portion includes, at a portion thereof for surrounding the heart, an expandable/contractable balloon that is located between the wire, which constitutes the annular portion, and the heart.

5. A cardiac treatment apparatus according to claim 1 or 2, wherein the annular portion is provided with a membranous portion that can be deformed into a shape that allows the membranous portion to pass through the port and that can be deformed to be expanded into a shape in which the membranous portion is brought into close contact with the ventricular apex when the annular portion is located at the position for surrounding the outer circumference of the heart.

6. A cardiac treatment apparatus according to one of claims 1 to 5, further comprising a suction portion that can be deformed into a shape which allows the suction portion to pass through the port, that is located at a position where the suction portion is attached by suction to the sidewall of the heart having been moved by being pressed by the annular portion, and that can fix the position of the heart.

7. A cardiac treatment apparatus comprising:
an annular portion including a wire that can be deformed between a contracted shape that allows insertion into a cardiac sac via a tubular port disposed passing from a body surface to a pericardium and an annular expanded shape in which the annular portion is expanded in the cardiac sac, is fitted onto the outer circumference of the heart from a side of a ventricular apex, and is located at a position for surrounding the outer circumference of the heart;
a suction portion that is provided in the annular portion, that can be deformed into a shape which allows the suction portion to pass through the port, that is located at a position where the suction portion reaches the ventricular apex while the annular portion is being fitted onto the outer circumference of the heart from the side of the ventricular apex, and that is attached to the ventricular apex by suction; and
a pulling portion that pulls the suction portion in a direction intersecting a straight line connecting a mediastinum and the ventricular apex.
